# EUROPEAN PATENT SPECIFICATION

(11) **EP 3 556 214 B1**
(45) Date of publication and mention of the grant of the patent: **17.03.2021**
(21) Application number: 19170355.2
(22) Date of filing: 19.04.2019
(51) Int. Cl.: A01N 59/16, A01N 59/20, A61K 31/30, A61K 31/315, A61P 31/00

(54) **METHODS FOR THE PREVENTION OF INFECTIONS OF EPITHELIAL CELLS IN AN ANIMAL**
VERFAHREN ZUR PRÄVENTION VON INFEKTIONEN VON EPITHELZELLEN BEI EINEM TIER
PROCÉDÉS POUR LA PRÉVENTION D'INFECTIONS DE CELLULES ÉPITHÉLIALES CHEZ UN ANIMAL

(30) Priority: 19.04.2018 NL 2020799
(43) Date of publication of application: 23.10.2019
(73) Proprietor: Intracare B.V., 5466 AZ Veghel (NL)
(72) Inventor: Vulders, Carly, 5466 AZ Veghel (NL)
(74) Representative: De Clercq & Partners

(56) References cited:
- EP-A1- 2 724 724
- EP-A1- 3 001 904
- WO-A1-00/62609
- WO-A1-2004/003121
- WO-A1-2005/044287
- WO-A1-2017/191453
- US-A1- 2015 306 062
- A. RELUN ET AL: "Effectiveness of different regimens of a collective topical treatment using a solution of copper and zinc chelates in the cure of digital dermatitis in dairy farms under field conditions", JOURNAL OF DAIRY SCIENCE, vol. 95, no. 7, 1 July 2012 (2012-07-01), pages 3722-3735, XP055095261, ISSN: 0022-0302, DOI: 10.3168/jds.2011-4983

## Description

### FIELD OF THE INVENTION

The present inventions relates to methods for the prevention of infections of epithelial cells including the use of metal chelate formulations.

### BACKGROUND OF THE INVENTION

Farm animals such as swine, sheep, goats, horses, and cattle are prone to infectious diseases of the mammary gland, such as mastitis, and of the skin, such as exudative epidermitis, digital dermatitis (hairy hoof warts), hoof rot (interdigital phlegmon), stable hoof rot (interdigital dermatitis), and udder cleft dermatitis. The environment of these animals often promotes the development of pathogenic bacteria causing these infections. For instance, when a cow lays down after milking, some milk may leak from the teats, creating the ideal breeding ground for the pathogenic bacteria.

These conditions cause stress to the animals and can affect weight gain, reproductive rates, milk production (in the case of dairy cattle), and wool production (in the case of sheep).

Skin disease in a swine herd can cause a significant decrease in growth rate and feed efficiency. Hoof ailments in milk cattle may result in reduced milk production, reduced fertility, and increased risk of mastitis. Furthermore, skin ailments demand much more work and more treatment costs often leading to premature removal of the affected animals.

Exudative epidermitis is caused by Staphylococcus hyicus and is most commonly observed in piglets of 1-8 weeks of age. Lesions can progress from the face to the rest of the body and become strongly exudating. The only known treatment is with antibiotics.

Foot rot or hoof rot is a bacterial infection of the soft tissue between the two toes of the animal. It is very painful and contagious quickly spreading and affecting the whole herd. The bacteria causative of foot rot are common to the environment in which the animals live.

Usually, there is an injury to the skin between the hooves that allows the bacteria to infect the animal. Foot rot may also be promoted by high temperatures and humidity, causing the skin between the hooves to crack and let the bacteria infect the foot. The first signs of hoof rot are limping, holding limbs above the ground, grazing on knees, and reluctance to walk.

Animals with foot rot might show interdigital inflammation, fever, loss of appetite and accompanying weight loss, and develop mild to severe lameness with hoof deformity.

Animals with chronic infections show a loss of body condition and decreased production, resulting in an unhealthy animal overall.

Udder cleft dermatitis is skin disease commonly observed in dairy herds, particularly in heifers. However, the disease can be present at all stages of lactation, and even during non-lactating periods. Characteristic features of udder cleft dermatitis are a moist appearance and necrosis of the skin, and a foul odor. Lesions are mostly located bat the cranial edge of the cleft between the two cranial quarters, although it can also be centered between all four quarters.

Digital Dermatitis, also known as "Papillomatous digital dermatitis" (PDD) or "Mortellaro's Disease", "strawberries", "hairy foot warts", "Interdigital Papillomatous", "strawberry Heel", or "Italian foot rot" is an infectious claw disease attributed to bacterial infection. In the last years it has become more and more common in livestock farming. This disease can be recognized by an inflammation of the skin in the area where skin changes into claw horn. The inflammable spot is reddish and looks a bit like a strawberry. Most often the hairs are standing upright around the affected areas

Interdigital dermatitis (not be confused with hoof rot), or stable hoof rot, is a chronic inflammation of the skin in the area between the toes of the feet (interdigital cleft). This infection is caused by the bacterium Treponema. The skin in the area of the interdigital cleft appears puffy with a dry exudation causing a crust to form.

Infection of the milk-secreting epithelial cells of the mammary gland, also known as mastitis, is recognized as the most costly disease in dairy cattle, as tissue damage reduces the number and activity of epithelial cells and consequently contributes to decreased milk production. Diagnosis of clinical mastitis may be based on the appearance of abnormally appearing milk (e.g. watery, clots, bloody). The amount of swelling of the udder, the severity of pain and the overall appearance of the cow may indicate the severity of infection.

The infected animals are preferably separated away from the herd as soon as possible to prevent the infection from spreading. Treatment of infectious diseases of the mammary gland or of the skin often consists in the use of antimicrobial products, such as antibiotics.

The milk of dairy cattle being subjected to a systemic antibiotic treatment is not marketable until all antibiotic residues have left the cow's system, leading to great economic losses for the dairy farmers. Furthermore, the use of antibiotics does not reduce the incidence of the infectious diseases of the mammary gland and/or of the skin.

In an attempt to inhibit bacterial growth, powders with drying and/or disinfecting properties are applied. In theory, this approach may inhibit microbial growth, but in practice the use of these powders has several disadvantages including dust formation, which may be harmful to inhale and pollutes the barn, dry and irritate the skin of the farmer and the udder (some products even have to be covered with bedding material in order not to come into contact with the animal skin), low cost-efficacy as the powders may only work if the bedding is already clean and dry; the often uneven distribution in the cubicles (as there are often spots without powder) and the fact that the powders may clump together, thereby physically damaging e.g. the claw skin.

Therefore, a need exists for methods for the prevention of infectious diseases of the mammary gland, in particular mastitis, and the skin that are effective and convenient.

### SUMMARY OF THE INVENTION

Provided herein are methods involving the use of formulations comprising copper and zinc chelates for the prevention of infections or reducing the risk of epithelial cells in animals, more particularly in animals, more particularly animals kept in a housing. The applicants have surprisingly found that compositions which have been previously described to be able to treat infections of epithelial cells in an animal by administering topically to the animal, are equally effective as a preventive measure for infections of epithelial cells, and particularly mastitis, exudative epidermitis, digital dermatitis (hairy hoof warts), hoof rot (interdigital phlegmon), stable hoof rot (interdigital dermatitis), and udder cleft dermatitis when applied on the housing of the animal. While spraying of the housing of animals with disinfectant in between uses is common, there is no suggestion in the art to prevent infections of epithelial cells by applying the specific compositions of the invention to the housing of the animals.

More particularly, provided herein are methods for prevention or reducing the risk of infections of epithelial cells in an animal and conditions related to infections of epithelial cells in an animal in an animal, comprising the step of applying on one or more interior surfaces of the housing of said animals a composition comprising copper and zinc chelates, wherein said copper and zinc chelates are suspended and optionally micronized in a liquid.

In particular embodiments, the composition comprising copper and zinc chelates is applied on one or more interior surfaces of said housing coming into contact with said animals.

In particular embodiments, the composition comprising copper and zinc chelates is applied on the flooring of said housing, more particularly to the bedding provided thereon.

In particular embodiments, the application of the composition comprising copper and zinc chelates on said one or more interior surfaces of said housing is achieved by spraying.

In particular embodiments, the composition is characterized in that the total amount of copper and zinc chelate in said formulation ranges from 1 to 99,99%. In particular embodiments, the composition comprises between 40% and 60% metal chelates, such as between 30 and 70% copper chelate and between 5 and 20 and zinc chelate. In further embodiments, the composition comprises between 1 to 25 percent copper and zinc chelates by weight.

In particular embodiments, the chelate is selected from EDTA, DTPA, HEDTA, MGDA, GLDA. In particular embodiments, said copper chelate is selected from copper EDTA, copper DTPA, and copper HDPA; and said zinc chelate is selected from zinc EDTA, zinc DTPA, and zinc HDPA. In particular embodiments, said copper and zinc chelates are potassium, diammonium or dialkalimetal salts of copper(EDTA) and zinc(EDTA).

In particular embodiments, said dialkalimetal salts are the disodium salts of copper(EDTA) and zinc(EDTA). In particular embodiments, said copper and zinc chelates are suspended in a water-based solution.

In particular embodiments, said micronized copper and zinc chelates are suspended in a non-aqueous liquid. In particular embodiments, said liquid is selected from propanol, isopropanol, butanol, isobutanol, or a mixture thereof, preferably said liquid is propanol.

In particular embodiments, the copper chelate: zinc chelate molar ratio of said copper and zinc chelates ranges from 5:1 to 1:2. In further embodiments the copper chelate: zinc chelate molar ratio of said copper and zinc chelates ranges from the 2 : 1 to 1 : 2.

In particular embodiments, said composition further comprises a silicate.

### BRIEF DESCRIPTION OF DRAWINGS

The following description of the figures of specific embodiments of the invention is merely exemplary in nature and is not intended to limit the present teachings, their application or uses.
Figure 1: Growth of 5 different bacteria species on culture plates containing increasing concentrations of chelated copper.
Figure 2: Percentages of elevated (A) and new (B) cases of high cell counts between year 0 (i.e. the year prior to start of treatment; grey bars) and year 1 (black bars)
Figure 3: Data and boxplots of cell count in spray group and control group on 2 combined timepoints during the trial.
Figure 4: Comparison of the individual cell counts of all animals during the milk control before and after 4 weeks of spraying

### DETAILED DESCRIPTION OF THE INVENTION

The term "about", when used in relation to a numerical value, has the meaning generally understood in the relevant art. In certain embodiments the term "about" may be left out or may be interpreted to mean the numerical value +10%; or +5%; or +2%; or +1%.

Whenever used herein in relation to a percentage, w/w means weight/weight and w/v means weight/volume.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.

Furthermore, the terms first, second, third and the like in the description and in the claims, are used for distinguishing between similar elements or steps and not necessarily for describing a sequential or chronological order, unless specified. It is to be understood that the terms so used are interchangeable under appropriate circumstances and that the embodiments described herein are capable of operation in other sequences than described or illustrated herein.

Reference throughout this specification to "one embodiment" or "an embodiment" means that a particular feature, structure or characteristic described in connection with the embodiment is included in at least one embodiment envisaged herein. Thus, appearances of the phrases "in one embodiment" or "in an embodiment" in various places throughout this specification are not necessarily all referring to the same embodiment, but may.

Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The inventors have found that by applying a composition comprising copper and zinc chelates as described herein on one or more interior surfaces of the housing of animals the onset and/or the worsening of infections of epithelial cells in an animal and conditions related to infections of epithelial cells could be prevented. The methods and compositions of the invention are particularly useful for animals kept in housing, such as, but not limited to farm animals. These include diseases of epithelial cells in farm animals such as cattle, pigs, sheep, goats, poultry etc., Examples of diseases of epithelial cells include, but are not limited to mastitis in cattle or pigs, exudative epidermitis, digital dermatitis (hairy hoof warts), hoof rot (interdigital phlegmon), stable hoof rot (interdigital dermatitis), pododermatitis, foot rot and udder cleft dermatitis. The application of the composition comprising copper and zinc chelates on surfaces of the animal housing is particularly of interest for preventing the onset and/or the worsening of infections in lactating animals, such as mastitis. Additionally it is envisaged to be useful for the treatment and/or prevention of epithelial diseases in other livestock, such as the diseases listed above.

The compositions and methods as envisaged herein prevent bacteria from taking hold and causing infection, and keep the epithelial cells of the animal in top condition. As the methods as envisaged herein relate to applying the composition on the interior surfaces of the housing of animals, the animal farmer does not have to apply the composition to each animal individually, thereby saving time and effort. In addition, as the animals are not being handled by humans to bring the composition into contact with the animals, this reduces the stress to the animals. Moreover, infections of epithelial cells of animals often result from micro-organisms present on the interior surfaces of the housing of said animals and/or skin lesions may occur by coming into contact with the interior surfaces of the housing of said animals. Accordingly, the method for prevention as envisaged herein allows addressing the possible onset or worsening of an infection of the epithelial cells of the animal immediately, before signs of such an infection would be visible or noticeable for the animal keeper. The copper and zinc chelates as described herein are dissolved or suspended in a liquid, which prevents dust formation and dirty hands while handling the chelates. Furthermore, the copper and zinc chelates as described herein can be applied on the one or more interior surfaces of the housing by spraying, which is easy, saves time and effort and allows an even distribution of the copper and zinc chelates. Unlike some known powders with drying and/or disinfecting properties which are used for the prevention of infection of epithelial cells of animals, and conditions associated with or resulting from such infections (e.g. lime, Actisan® (Timac Agro), Stalosan F® (MS Schippers)), the composition comprising copper and zinc chelates as described herein, is friendly to the skin of the animal and it does not cause irritation. Additionally, it promotes healing of the raw and/or irritated skin.

The present application relates to methods for the prevention of infection of epithelial cells of animals, and conditions associated with or resulting from such infections, and specifically for the prevention of mastitis. More particularly, present application relates to methods for the prevention of the onset or the worsening of infection of epithelial cells of animals, and conditions associated with or resulting from such infections, preferably for the prevention and/or treatment of mastitis.

The term "prevention" as used herein, refers to prophylactic or preventive measures, wherein the aim is to prevent or lessen the chances or risk of incidence of an undesired affliction, such as to prevent occurrence, development and progression of infection of epithelial cells of animals, and conditions associated with or resulting from such infections.

Beneficial or desired clinical results may include, without limitation, absence or alleviation of one or more symptoms or one or more biological markers, diminishment of extent of disease, stabilised (i.e., not worsening) state of disease, delay or slowing of disease progression, and the like. More particularly, in the context of the present invention, the biological marker may be the absence or reduced occurrence of clinical symptoms of epithelial infections.

The term "infection of epithelial cells of animals", as used herein, refers to infections of any epithelial cell, but more particularly the skin, the milk secreting epithelial cells of the mammary gland and the hooves of animals. In particular embodiments, the infections are or include bacterial infections, for example a *Staphylococcus Aureus* infection. These infections include but are not limited to greasy pig disease (exudative epidermitis), porcine cutaneous spirochetosis, porcine necrotic ear syndrome, hairy hoof warts or Mortellaro's Disease (digital dermatitis), hoof rot (interdigital phlegmon), stable hoof rot (interdigital dermatitis), udder cleft dermatitis and infection of the mammary gland (infection of the milk secreting epithelial cells of the (lactating) mammary gland), foot rot in sheep, pododermatitis in poultry, pig ear/tail biting, pig wound infection after castration, cattle (calf) wound infection after dehorning, calf/pig navel infection after birth, infection of post-operative wounds, secondary infection of acute trauma (wounds), infection of cannibalism (picking) wounds of birds/poultry, infection of sores, coronary band infection (pigs), infection of intertriginous dermatitis (or intertrigo), necrotic dermatitis (or foul udder) in cattle and pigs.

In particular embodiments, the methods envisaged herein are used for the prevention of M1 (early stage) and/or M2 (ulcerative stage) lesions on the skin or in the hoof. The term is also meant to cover conditions associated with or resulting from epithelial cell infections, in particular irritation (including reddening, swelling and other phenomena), pain, inflammation, more in particular skin or mammary gland inflammation.

In particular embodiments, the "conditions related to epithelial cell infections" include reduced growth, reduced reproductive rates, and in the case of sheep, reduced wool production, less milk production, increased risk of mastitis-associated inflammation, as well as those conditions further mentioned herein. The conditions may be acute as well as chronic.

The present application generally refers to the prevention and treatment of infections by "pathogens" or "micro-organisms". Different pathogens are envisaged to be targeted in the context of the present invention and these include, but are not limited to *Escherichia coli* (E. coli), *Streptococcus uberis, Staphylococcus aureus,* and a wide variety of other contagious and environmental organisms have been identified as potential pathogens causing infections of epithelial cells in animals, more particularly in farm animals.

As indicated above, the methods envisaged herein are in particular suitable for the prevention of infection of the milk secreting epithelial cells of the mammary gland (mastitis).

The term "mastitis" as used herein refers to the microbial infection of the milk-secreting epithelial cells of the mammary gland and the resulting inflammatory reaction of the mammary gland that is usually caused by a microbial infection of the milk-secreting epithelial cells. The infection occurs after bacteria gain entrance to the mammary gland via the teat canal. The onset and development of mastitis may be evaluated by visualization and palpation of the udder, visualization of the milk, detection of somatic cells by California Mastitis Test (CMT) or determining the electrical conductivity of the milk.

The (somatic) cell count is an indicator of the quality of milk-specifically, its low likeliness to contain harmful bacteria, and thus its high food safety. White blood cells (leukocytes) constitute the majority of somatic cells in question. The number of somatic cells increases in response to pathogenic bacteria like *Staphylococcus aureus,* a cause of mastitis. The cell count is quantified as cells per milliliter. General agreement rests on a reference range of less than 100,000 cells/mL for uninfected cows and greater than 250,000 for cows infected with significant pathogen levels.

In particular embodiments, the methods envisaged herein prevent the microorganism, more particularly the bacteria, to enter the mammary gland via the teat canal.

Although the compositions envisaged herein may be applied to one or more interior surfaces of the housing of any (ungulate) animal, the application to one or more interior surfaces of the housing of farm animals, more particularly mammals such as sheep, goats, horses, pigs and cattle is particularly envisaged. In particular embodiments, the (farm) animals are lactating animals, preferably lactating cattle.

In particular embodiments, the method envisaged herein is applying the composition on the interior surfaces of the housing coming into contact with said animals, and especially those surfaces of the housing coming into contact with the animal's body zones or surfaces susceptible of infection and/or lesions. Non-limiting examples of such animal body areas, zones or surfaces are the knuckles; the genitals, the skin, the claws, the hoofs, and the udder, the legs, and more generally, the extremities of the animal. When referring to surfaces "coming into contact" with the animal's body it is intended to refer to surfaces of the housing which, under normal circumstances of breeding, will come into contact with the animal's body. Examples of housing include but are not limited to barns, stables, stalls, pens, hutches, farrowing or gestation crates or cages. The surfaces are typically treated with the compositions of the present invention at moments when the animal's body is not in contact with the surface, more preferably when the animal is not present in the housing.

The methods envisaged herein are particularly suitable for the surfaces of the housing coming into contact with said animals when the animals lay down, more particularly the flooring of the animal housing. Animals lying down on flooring on which the composition envisaged herein is applied, enables a longer contact-time between the animal's body areas, zones or surfaces suffering from or susceptible of infection and/or lesions, such as the knuckles or udder and the compositions envisaged herein. Of particular interest is the flooring of the designated laying areas of said animals.

The flooring may be any type of flooring suitable for animal houses, including, but not limited to, bare-earth, gravel, metal grids, concrete, bedding material, and stall mats. The methods envisaged herein are in particular suitable for substantially smooth and water cleanable flooring surfaces, such as concrete or stall mats.

In particular embodiments, the flooring comprises bedding material, such as straw, sawdust, wood shavings, paper, compost, gypsum or sand. In particular embodiments, the bedding material is processed (dried and sterilized) manure, also referred to as dried manure solids (DMS). In more particular embodiments, the bedding material has itself a moist absorbing capacity (e.g. sawdust, wood shavings, paper, compost, sand) and/or is supplemented with a substance having a moist absorbing capacity (e.g. lime powder). The moist absorbing capacity will be highly dependent on the source (e.g. type of wood sawdust, way of processing/preparation of the bedding material, etc).

In particular embodiments, the one or more interior surfaces of the housing of the animals are cleaned with water and/or one or more disinfectants before applying the composition as described herein.

In particular embodiments, the application method envisaged herein is spraying the composition on one or more interior surfaces of the housing of animals.

This implies that the compositions envisaged herein are typically provided as sprayable compositions, meaning that they can be applied using a suitable spraying device. For example the viscosity will be sufficiently low, e.g. below 40 mPa.s, such as below 10 mPa.s.

In any given case, the quantity of the formulations of zinc and copper chelate to be applied and the frequency of application on the one or more interior surfaces of the housing of animals will be adjusted with the purpose to be attained (prevention of onset or prevention of worsening), the nature of the subject, the condition under consideration as well as the severity of the condition, the type of the formulation concerned, and any other relevant facts that may modify the activity of the zinc and copper chelate or the response of the subject, as is well known by those skilled in the art.

The formulations comprising copper and zinc chelates as envisaged herein may be applied to the one or more interior surfaces of the housing of animals once weekly or multiple times weekly, for example two, three, four, five, or six times per week or more, preferably twice weekly. In particular embodiments, it is envisaged to apply the formulations once daily or multiple times daily, for example two, three, or four times per day. In particular embodiments, at least two applications are envisaged within a time period of one week. In further particular embodiments, two applications are envisaged separated by 2 days (i.e. day 0 and day 3).

The amount of liquid used for spraying the housing of an animal will depend on the size of the animal and its housing. Typically, however, given that the liquid is sprayed, a large surface can be treated with a limited amount of liquid. Thus, the disadvantage of using a water formulation (e.g. that it provides further humidity to the bedding) can be minimized.

The size of the housing or the surface of the bedding is of course dependent on the size of the animal. The surface of the housing of a cow is typically about 1.70-1.90x1.15-1.40m. It is moreover noted that it may not be necessary to treat the entire housing or even the entire surface of the bedding of said housing. In particular embodiments, such as where the housing of cattle is being treated, only the area where the udders of the cow need to be treated, which corresponds to about a 1m area at the rear end (where of the housing. Where the animal is a pig, the surface of the housing will typically be at least about 1.3m². Broilers are typically kept with 12-18 on a surface of about 1 m², but in these embodiments the totality of the floor of the housing will need to be sprayed. Irrespective of the above, the compositions of the inventions allow the treatment of the housing of the animals in a much more efficient way. For instance, it is envisaged that only about 10 ml liquid volume is required to ensure the coverage of the relevant part of the bedding of a cow. In this way 1 liter spray replaces 10 kg of commonly used powder. This is beneficial for transport, storage, and use.

Suitable chelating agents or for forming copper and zinc chelates are known in the art and include ethylenediaminetetraacetic acid (EDTA), pentetic acid or diethylene triamine pentaacetic acid (DTPA), 2,2'-dipyridylamine (HDPA), N-(hydroxyethyl)-ethylenediaminetriacetic acid (HEDTA), methylglycinediacetic acid (MGDA), L-glutamic acid-N,N-diacetic acid (GLDA) and the like. In particular embodiments, the copper chelate is selected from copper EDTA, copper DTPA, and copper HDPA; and the zinc chelate is selected from zinc EDTA, zinc DTPA, and zinc HDPA. The skilled person will understand that copper and zinc ions may form charged complexes with the (conjugate bases of) the chelating agents, and that the complexes may be provided with counterions such as alkalimetal ions to obtain neutral chelates.

In particular embodiments, the copper and zinc chelates are EDTA chelates of copper and zinc, in particular the salts of EDTA chelates of copper and zinc. EDTA chelates are widely available and are well tolerated by the skin.

In particular embodiments, the copper and zinc chelates are salts consisting of two monovalent cations and copper[EDTA]²⁻ and of Zn[EDTA]²⁻.

In particular embodiments, the copper and zinc chelates are salts of copper[EDTA]²⁻ and of Zn[EDTA]²⁻. In particular embodiments, the salts are copper diammonium EDTA and/or zinc diammonium EDTA. In further embodiments the salts are copper potassium EDTA and/or zinc potassium EDTA.

The copper and zinc diammonium EDTA salts have the advantage that they are soluble in water. It is advantageous to have the chelates dissolved rather than suspended to avoid sedimentation/uneven distribution and clogging of the sprayer. While adding water to the bending may be disadvantageousas only a limited amount of water is required (e.g. 10 ml), this does not affect the quality of the bedding.

Of further interest are the dialkalimetal salts of copper[EDTA]²⁻ and of Zn[EDTA]²⁻. Of further interest are the disodium salts of copper(EDTA) and of Zn(EDTA) complex. These salts may also be referred to as copper and zinc disodium ethylenediaminetetraacetic acid or ethylenediaminetetraacetate-copper- and zinc-sodium complex. The disodium salts of copper[EDTA]²⁻ and of Zn[EDTA]²⁻ in particular are attractive due to their effectiveness, thereby reducing amount of copper and zinc to be applied, and reducing concomitant side effects in animals when they come into contact with the treated housing surface(s).

In the formulations for use in the methods envisaged herein, the copper and zinc chelates are dissolved or suspended in a liquid. Non-limiting examples of suitable liquids are water or evaporative liquids such as propanol, isopropanol, butanol and isobutanol.

In particular embodiments, the fluid serves merely as a carrier fluid and/or evaporates easily leaving behind the particles on the area of interest.

In particular embodiments, the chelates may be suspended in the form of micronized particles in a skin-tolerable liquid e.g. non-toxic, causing no irritation or allergic reactions. In particular embodiments the liquids are evaporative (volatile) and skin-compatible. The term "evaporative liquid" or "volatile liquid" as used herein refers to a liquid having a vapor pressure higher than the vapor pressure of water at a temperature of about 25°C.

The liquid in which the micronized particles are suspended is selected such that substantially none of the copper or zinc chelate dissolves, the term substantially none meaning that no or a small amount of the copper and zinc chelates present in the formulation dissolves, e.g. less than 5%, or less than 2 %, or less than 1 % (each % being w/v). Said liquid is non-aqueous, although minor amounts of water, e.g. less than 5 %, or less than 2 %, or less than 1 % (each w/v) can be present.The formulations comprising evaporative liquids envisaged herein typically contain at least 45 w% of evaporative liquids, preferably at least 50 w%, and more preferably at least 60 w%, based on the total weight of the formulation excluding optional propellants.

Exemplary evaporative liquids that can be used include alcohols, in particular alkanols such a propanol, isopropanol, butanol, isobutanol, including mixtures thereof. They may contain small amounts of water, i.e. such amounts of water that do not cause the micronized particles to dissolve. In particular embodiments, the liquid comprises less than 20% (w/w) water, more preferably less than 10% (w/w) water, even more preferably less than 5% (w/w) water, and most preferably less than 1 % (w/w) water. In certain embodiments, such as when the chelates are suspended in the form of micronized particles, the liquid is free from water.

In alternative embodiments, the liquid present in the composition comprise copper and zinc chelates as described dissolved and/or suspended in water. The water is preferably purified water, such as distilled water or deionized water.

The total amount of the chelates suspended in the formulation for use in the methods envisaged herein may range from 1-80 per cent by weight, based on the total weight of the formulation, depending on the type of liquid as well as on the type of copper and zinc chelates.

In particular embodiments, such as when the chelates are suspended in the form of micronized particles, the total amount of copper and zinc chelate in the formulations for use in the methods envisaged herein, is maximally 30% by weight and typically ranges from 1-25 per cent by weight, based on the total weight of the formulation. In further particular embodiments the total amount of copper and zinc chelate ranges from 2-20 per cent by weight, from 5-20 per cent by weight, more particularly from 10-20 per cent by weight. In further particular embodiments the concentration is about 15 per cent by weight. In certain embodiments the total amount of copper and zinc chelate ranges from 1-25 per cent by weight, based on the total weight of the formulation excluding optional propellants. In further embodiments, the total amount of copper and zinc chelate ranges from 10-20 per cent by weight, more particularly about 15 per cent by weight, based on the total weight of the formulation excluding optional propellants.

In particular embodiments, such as when the chelates are suspended in the form of micronized particles, the copper and zinc chelates may be present in a copper chelate : zinc chelate molar ratio that is in the range of about 2 : 1 to about 1 : 2, or about 1.5 : 1 to about 1 : 1.5, the molar ratio between these chelates preferably being about 1 : 1.ln alternative embodiments, d the copper and zinc chelates are suspended or dissolved in a liquid, such as water. In these embodiments, the total amount of copper and zinc chelate in the formulations for use in the methods envisaged herein, ranges from 50-80% by weight, and is preferably around 70% by weight. In particular embodiments, the composition comprises between 45 and 55% (w/w) copper chelate, such as 47% (w/w) and between 5% and 15% of zinc chelate, such as 10-12% (w/w).

The zinc or copper chelates can be present in the formulations envisaged herein in a micronized or non-micronized form. The non-micronized formulations have the advantage that they are easier to produce.

In particular embodiments, the micronized zinc or copper chelates are present in the formulations envisaged herein as microparticles. In one embodiment, the mean surface weighed diameter is in the range of about 0.5 to about 75 µm, about 0.5 to about 50 µm, about 1 to about 50 µm, about 10 to about 30 µm, about 0.5 to about 10 µm, or about 1 to about 5 µm, or about 2 to about 4 µm, preferably about 1 to about 50 µm. For example, the mean surface weighed diameter may be about 20 µm. Or the mean volume weighed diameter is in the range of about 0.5 to about 75 µm, about 0.5 to about 50 µm, about 1 to about 50 µm, about 10 to about 30 µm, about 1 to about 10 µm, or about 2 to about 5 µm, or about 3 to about 5 µm. For example, the mean volume weighed diameter may be about 20 µm. The size or diameter of the microparticles can be determined as an equivalent spherical diameter via techniques such as Dynamic Light Scattering. Microparticles of the copper and zinc chelates provide the advantage that they can be sprayed. Indeed, providing the chelates as microparticles may allow for obtaining compositions having a high chelate content, which still have a suitable viscosity for spraying. Moreover, the ratio between surface and content is such that the particle will easily dissolve on the moist skin. However, in alternative embodiments, the compositions of the present invention may be applied by other means such as by flushing, brushing or mopping.

In particular embodiments, the microparticles have a size of about 20 µm. The span of the microparticle distribution may be in the range of about 75 to about 0.1, or about 50 to about 0.1, preferably in the range of about 3 to about 0.1, in particular about 3 to about 1, or about 2 to about 1, and more preferably about 1.5 to about 1.7.

The particles of the chelates are preferably present as a monomodal distribution.

Each of the copper and zinc chelates in micronized form can be prepared separately or they can be prepared in combination. When prepared separately, the micronized powders or micronized dispersions can be mixed subsequently. Also end formulations or intermediate formulations with only copper chelate and zinc chelate can be prepared and subsequently mixed.

The copper and zinc chelates in micronized form can be prepared using techniques known in the art. Such methods include milling, bashing and grinding. The mechanical means applied to reduce the effective average effective particle size can include a ball mill, an attritor/attrition mill, a vibratory mill, a planetary mill, media mills, such as a sand mill and a bead mill.

The micronized copper and zinc chelates can also be prepared by wet milling of the starting copper and zinc chelates as a dispersion in a suitable liquid medium in which they are essentially insoluble, for example in any of the skin-tolerable non-aqueous liquids mentioned herein.

A bead mill can be used with ceramic or metal beads. The milling can be done in a one step or multistep procedure, where in the latter instance different mills and/or beads can be used.

Prior to milling, the particle size of the material may be reduced first reduced to a particular size, e.g. a size of less than about 100 µm, which after sieving may be processed further.

The formulations for use in the methods envisaged herein, may contain other therapeutically active ingredients such as antibiotics, including antibacterial and antifungal compounds. In particular embodiments however, the compositions do not comprise antibiotics, more particularly they do not comprise specific antibiotics.

In alternative embodiments, the copper and zinc chelates are present in the formulations as described herein in a non-micronized form. For example, when the liquid in the formulation as described herein consists of water, the copper and zinc chelates may be dissolved in the water and thus be present in the formulations as described herein in a non-micronized form.

In particular embodiments the formulations for use in the methods envisaged herein comprise copper and zinc chelates as the only chelates present in the formulation. In further particular embodiments the formulation comprises copper zinc disodium EDTA as the only active ingredient.

The formulations for use in the methods described herein may further contain adjuvants, such as fragrances, colorants (e.g. dyes), dispersants, preservatives, stabilizing agents, anti-agglomeration agents, ingredients that improve the spraying characteristics of the formulations, etc. The addition of dyes to the composition may facilitate the identification of the surface areas of the housing treated with the composition and/or the skin surfaces which have been in contact with the treated surface areas of the housing. Suitable dyes for use in the composition include but are not limited to Brilliant blue FCF, Patent blue V, and curcumin.

In certain embodiments, the compositions comprise between 0.010 w% and 1.0 w% of dyes, more particularly between 0.050 w% and 0.50 w%.

In particular embodiments, the composition comprises one or more fragrances, preferably fresh fragrance such as peppermint, eucalyptus and/or menthol. In certain embodiments, the compositions comprise between 0.10 w% and 2.0 w% of one or more fragrances. For example, the composition comprises 1.0 w% of one or more fragrances. The presence of one or more fragrances in the composition allows obtaining a pleasant smell in the housing of the animal. Suitable fragrances are known to the skilled person and include, but are not limited to essential oils. Examples of fragrances may include menthol, peppermint, eucalyptus etc.

In particular embodiments, the composition comprises one or more dispersants. Typically, the concentration of dispersants is less than 1%, such as between 0.5-0.8%. Suitable dispersants are known to the skilled person.

In particular embodiments, the compositions comprise one or more silicates, such as Bentone gel®. In certain embodiments, the compositions comprise between 0.1 w% and 2 w% of silicates.

The compositions may also contain ingredients that improve the spraying characteristics of the formulations or the color. The addition of dyes to the composition may facilitate the identification of the surface areas of the housing treated with the composition and/or the skin surfaces which have been in contact with the treated surface areas of the housing. Suitable dyes for use in the composition include but are not limited to Brilliant blue FCF, Patent blue V, and curcumin. In certain embodiments, the compositions comprise between 0.01 w% and 1 w% of dyes, more particularly between 0.05 w% and 0.5 w%.

In particular embodiments, the composition comprises:
- about 30 to about 70% (w/w), preferably about 50% (w/w), of copper diammonium chelate;
- about 5 to about 20 % (w/w), preferably about 10% (w/w), of zinc diammonium chelate;
- about 1 % (w/w) of one or more fragrances;
- about 0.8 % (w/w) of one or more dispersants; and
- up to 100% (w/w) of water.

In particular embodiments, the composition is an aqueous solution containing 39% water, 47% Copper diammonium EDTA and 12% zinc diammonium EDTA, 1% essential oils (peppermint, eucalyptus, methol) and <1% dispersing agent.

The formulations for use in the methods described herein can be applying on the one or more interior surfaces of animal housing by various devices, which may be sterile or non-sterile. Such devices comprise multi-dose or unit-dose containers or unit-dose sprays or any other container, including glass bottles with a spray device.

In particular embodiments, the formulation for use in the methods described herein can be applied on the one or more interior surfaces of animal housing by a low pressure hose or jet.

Preferably, the low pressure hose or jet is configured to operate with a pressure of at most 10 bar, at most 9 bar, at most 8 bar, at most 7 bar, at most 6 bar, at most 5 bar, at most 4 bar or at most 3 bar, more preferably at most 7 bar.

Using a spraying device significantly facilitates the application of the copper and zinc chelate formulation on the on one or more interior surfaces of the housing of said animals.

Furthermore, spraying of the copper and zinc chelate formulation allows an even distribution of the formulation on the one or more interior surfaces of the housing of said animals.

The application thus also provides for a low pressure sprayer such as a low pressure hose or jet comprising the formulations described herein. More particularly, the low pressure hose or jet is designed for spraying large surfaces (i.e. wider nozzle) or has a wide spray pattern, which typically implies a tight nozzle. In particular embodiments, the nozzle has a spray angle of at least 70°, 80°, 90°, 100°, 110, 120° or more.

Where the sprayer has different nozzle settings, the tight nozzle is typically used in the methods of the invention.

The following examples are meant to illustrate the present invention and should not be construed as a limitation of its scope.

### EXAMPLES

### Experiment 1: prevention of the onset and the worsening of mastitis in lactating cows

### A. Preparation of the composition

A composition comprising copper and zinc cations as active ingredients, in the form of copper disodium EDTA and zinc disodium EDTA, is made. The composition (including excipients such as propellants) comprises about 5 w% of each chelate.

The formulation further comprises a number of excipients. Isopropyl alcohol is used as a carrier liquid, and colloidal anhydrous silica is used as a rheological additive. Butane and propane are used as propellants. The formulation is used for the prevention of mastitis in a number of field trials, as discussed below.

### B. Treatment of the animal-housing of lactating cows with the formulation as described herein

During a period of 8 weeks, the formulation as described in section A is sprayed once a day on the flooring of designated laying areas for 5 lactating cows. A neighboring area is left untreated but handling of the cows is identical.

The development of mastitis in the lactating cows is evaluated weekly by
- Visualization and palpation of the udder: the udder of the lactating cows is checked for redness, hardness, swelling and being warm to touch; and

Visualization of the milk: at the time of milking, changes (e.g. presence of flakes or clots) in the milk are monitored.

### C. Results

Treatment of the flooring of the designated laying areas for lactating cows reduces the number of lactating cows suffering from mastitis, as well as the severity of the infection. This effect is not observed for the control condition.

No adverse effects are observed during or after treatment period. The treatment does not require a withdrawal time and does not interfere with milk quality.

### Experiment 2: Antimicrobial and skin healing effect of the compositions of the invention

The main mastitis-causing pathogens are *Escherichia coli* (E. coli), *Streptococcus uberis* and *Staphylococcus aureus,* and a wide variety of other contagious and environmental organisms have been identified as potential mastitis pathogens.

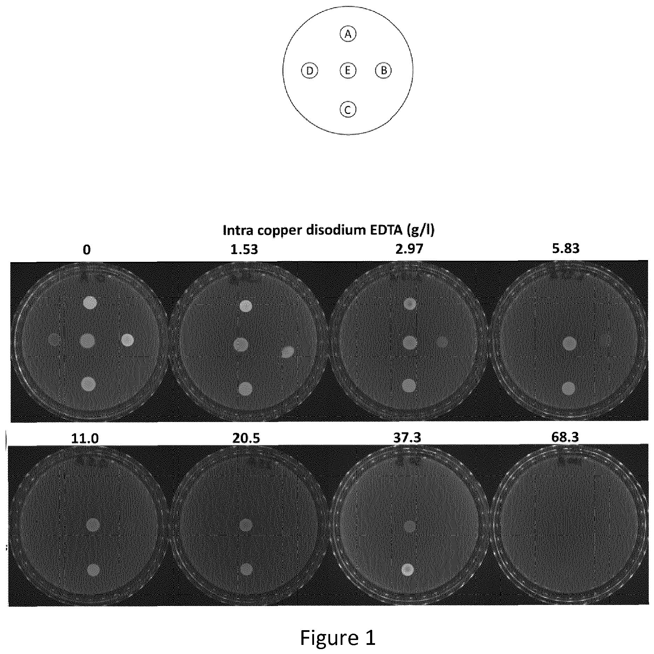

The bactericidal effect of chelated copper has been evaluated on 5 different micro-organisms including the mastitis associated E. coli and S. aureus. By growing of these bacteria on culture plates containing increasing concentrations of chelated copper, the minimal inhibitory concentration (MIC) of the copper chelate on these bacteria was determined (Fig. 1 and Table 1).

**Table 1. Minimal Inhibitory Concentration (MIC) of chelated copper on 5 bacteria species.**

| | Pathogen | mg chelated copper per ml product |
|---|---|---|
| A | *S*. *aureus* | 5.83 |
| B | *S*. *epidermidis* | 11.0 |
| C | *E. coli* | 68.3 |
| D | *S. pyogenes* | 1.53 |
| E | *P. auruginosa* | 68.3 |

### Experiment 3: prevention of the onset and the worsening of mastitis in lactating cows A. Preparation of the composition

A composition comprising
- 47% (w/w), of copper diammonium chelate ;
- 12% (w/w) of zinc diammonium chelate;
- 1 % (w/w) of one or more fragrances;
- About 0.8 % (w/w) of one or more dispersants; and
- up to 100% (w/w) of water,
wherein the copper and zinc diammonium chelates are dissolved or suspended in water, was prepared.

The formulation is used for the prevention of mastitis in a field trial, as discussed below.

### B. Trials

### Trial 1

**Table 2: details of trial 1**

| | |
|---|---|
| **Number of animals** | 150 |
| **Type of milking** | 2 Lely robots |
| **Type of bedding** | Chopped straw |
| **Use of powder b** | Actisan (See Annex 1) |
| **Initial cell count** | Around 75,000 |
| **Spray treatment** | 2x per week ( during time period Actisan is not used) |
| **Duration of trial** | 8 weeks |
| **Comparison** | 2 months new spray vs. preceding period with Actisan |

The development of mastitis in the lactating cows was evaluated weekly by Somatic Cell Count (SCC) of the bulk milk of the lactating cows. The cell count is quantified as cells per milliliter. General agreement rests on a reference range of less than 100,000 cells/mL for uninfected cows and greater than 250,000 for cows infected with significant pathogen levels

**Table 3. Cell count in the bulk milk.**

| **Date** | **Bulk milk count** | **Product** |
|---|---|---|
| Day 1 (October 18 year 1) | 75,000 | Actisan powder |
| Day 1 + 18d (November 5 year 1) | 71,000 | Actisan powder |
| Day 1 + 39d ( November 26, year 1) | 60,000 | Spray |
| Day 1 + 51d (December 8, year 1) | 73,000 | Spray |
| Day 1 + 86d (January 12, year 2) | 68,000 | Actisan powder |

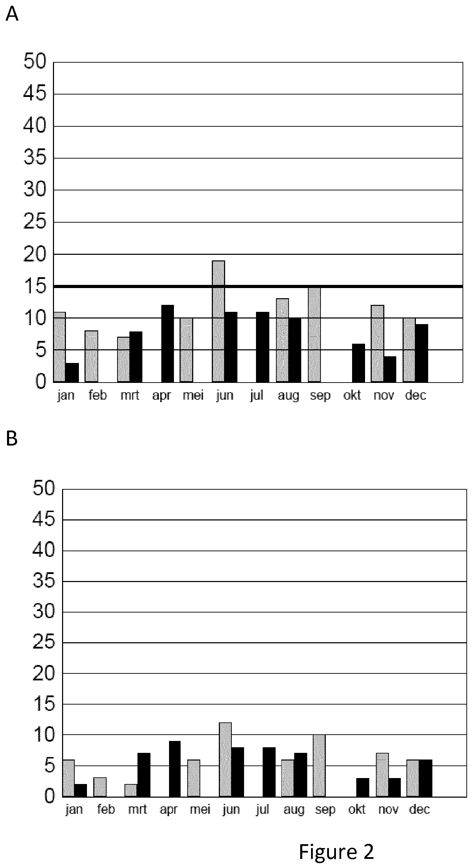

The milk of animals with a high cell count was separated and therefore, does not contribute to the bulk milk count shown in Figure 2. The figure provides the percentage of elevated (A) and new (B) cases of animals with a high cell counts between year 0 (grey bars) and subsequent year 1 (black bars). It can be seen that in the numbers remained stable after quitting the Actisan treatment and starting the spray treatment.

### Trial 2

**Table 4: details of trial 2**

| | |
|---|---|
| **Number of animals** | 100 |
| **Type of milking** | 2x6 milking parlour |
| **Type of bedding** | Rapeseed straw with premixed lime powder |
| **Use of powder** | Lime (premixed in the straw, so not able to quit), daily refreshment of bedding |
| **Initial cell count** | 100,000 - 125,000 |
| **Spray treatment** | 2x per week additional to the lime powder |
| **Start date trial** | 16 November 2018 |
| **Duration of trial** | 8 weeks |
| **Comparison** | Period before vs. period during spraying |

Bulk tank cell count was measured every 4 emptyings (approximately 2 times per month). 1 animal with mastitis, not different from usual.

Bulk tank milk cell count slightly increased from 100,000 - 125,000 to 150,000 after start of spraying, but stabilized afterwards around 125,000. No negative effects were observed with the spraying solution.

### Trial 3

In this trial a composition according to an embodiment of the invention was compared to the use of lime powder.

**Table 5: details of trial 3**

| | |
|---|---|
| **Number of animals** | 300 (in 3 different houses), compared 2 groups in same lactation phase. |
| **Type of milking** | Robot |
| **Type of bedding** | Oat/barley straw |
| **Use of powder** | Potassium lime (PROKA) 75$ per tons, ±45 tonnes per year for 250 cows |
| **Initial cell count** | Appr. 100,000 - 150,000 |
| **Spray treatment** | 1x spray/1x lime per week, control house 2x lime powder |
| **Start date trial** | 8 November 2018 |
| **Duration of trial** | 8 weeks |
| **Comparison** | 1x spray/1x lime group vs. 2x lime group |

The spraying was experiences as fast en easy, no inhaling of powders for the employee.

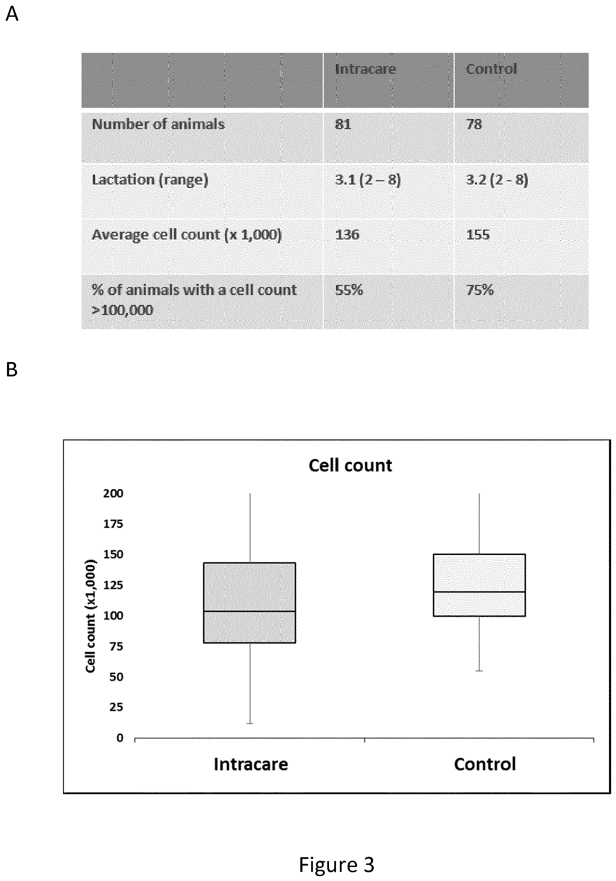

The average cell counts and the percentage of animals with a cell count above 100,000 was better for the spray group compared to the control group (Fig. 3). No negative effect of the spray was observed.

The compositions of the invention thus provide an interesting alternative for the prevention of epithelial diseases such as mastitis with many advantages compared to hygiene powders.

### Trial 4

**Table 6: details of trial 4**

| | |
|---|---|
| **Number of animals** | 300 (in 3 different houses), compared 2 groups in same lactation phase. |
| **Type of milking** | Robot |
| **Type of bedding** | Oat/barley straw |
| **Use of powder** | Potassium lime (PROKA) 75$ per tons, ±45 tonnes per year for 250 cows |
| **Initial cell count** | Appr. 100,000 - 150,000 |
| **Spray treatment** | 1x spray/1x lime per week, control house 2x lime powder |
| **Start date trial** | 8 November 2018 |
| **Duration of trial** | 8 weeks |
| **Comparison** | 1x spray/1x lime group vs. 2x lime powder group |

In order to obtain a clear view on the real effect of the composition of the invention, it was decided to switch the treatment and control house of trial 3 in a second trial period, meaning that the untreated house of trial 3 was now treated and the treated house of trial 3 now served as a control.

After 5 weeks, both houses were comparable, meaning that the composition of the invention is a good replacement of lime treatment.

### Trial 5

**Table 7: details of trial 5**

| | |
|---|---|
| **Number of animals** | 90 |
| **Type of milking** | traditional milking parlour |
| **Type of bedding** | Sawdust on water beds |
| **Use of powder** | Lava3 (lime) 2x per day (Annex 2) |
| **Initial cell count** | 200,000 |
| **Spray treatment** | Lava3 every morning, spray every evening (so 7x per week) |
| **Start date trial** | 25 January 2019 |
| **Duration of trial** | 10 weeks (up to second milk control) |
| **Comparison** | Period before vs. period during spraying (and preferably also after) |

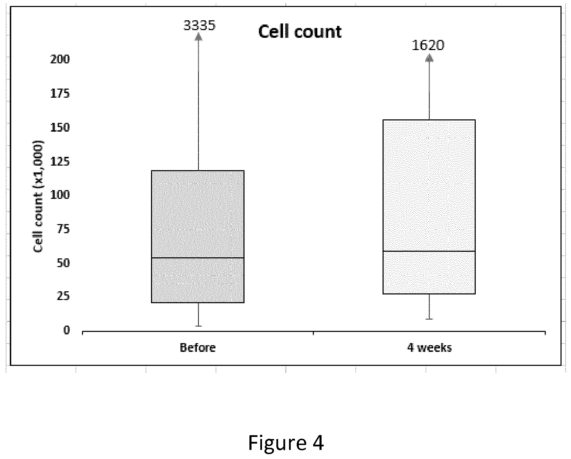

The results of the bulk milk tank cell counts are provided in Table 8 and the individual cell counts of the animals are provided in Figure 4. These indicate that both products appear to have a similar effect.

**Table 8:. Bulk milk tank cell counts**

| **Date** | **Bulk tank milk count** |
|---|---|
| Before trial start, January 9 | 198 |
| 9 february | 172 |
| 11 march | 166 |
| 20 march | 189 |

### Trial 6

**Table 9: details of trial 6**

| | |
|---|---|
| **Number of animals** | 90 |
| **Type of milking** | traditional milking parlour |
| **Type of bedding** | Sawdust 2x per day |
| **Use of powder** | Dekamix lime pH 12 daily |
| **Initial cell count** | >300,000 |
| **Spray treatment** | Lime 5x per week Spray 2x per week |
| **Start date trial** | 12 February 2019 |
| **Duration of trial** | 10 weeks (up to second milk control) |
| **Comparison** | Period before vs. period during spraying (and preferably also after) |

The bulk tank milk cell count indicated that the average of the counts before the trail start was 245 (193 - 360), while the average dropped to 214 (161 - 269).

In general, it can be concluded that the effect of the product of the invention is at least comparable if not improved compared to commonly used powders.

## Claims

1. A method for reducing the risk of epithelial infections in an animal kept in a housing, comprising the step applying a composition comprising copper and zinc chelates to one or more interior surfaces of said housing.

2. The method according to claim 1, wherein said copper and zinc chelates are suspended in a liquid.

3. The method according to claim 1, wherein aid copper and zinc chelates are micronized in a liquid.

4. The method according to any one of claims 1 to 3, wherein said composition comprising copper and zinc chelates is applied on the flooring or bedding covering said flooring of said housing.

5. The method according to any one of claims 1 to 4, wherein the application of the composition comprising copper and zinc chelates on said one or more interior surfaces of said housing is achieved by spraying.

6. The method according to any one of claims 1 to 5, wherein the total amount of copper and zinc chelate in said formulation is between 40% and 60%.

7. The method according to claim 6, wherein the composition comprises between 30% and 50% copper chelate and between 5% and 20% and zinc chelate.

8. The method according to any one of claim 1 to 7, wherein said chelate of said copper and/or zinc chelate is selected from EDTA, DTPA, HDPA, HEDTA, MGDA and GLDA.

9. The method according to any one of claim 1 to 8, wherein said copper and zinc chelates are disodium or diammonium salts of copper(EDTA) and zinc(EDTA).

10. The method according to any one of claims 1 to 9, wherein the copper chelate: zinc chelate molar ratio of said copper and zinc chelates ranges from 5:1 to 1: 2.

11. The method according to any one of claims 1 to 9, wherein said method is a method of preventing an epithelial infection selected from mastitis, exudative epidermitis, digital dermatitis, hoof rot, stable hoof rot, pododermatitis, foot rot and udder cleft dermatitis in said animal.

12. A composition comprising
- between 30 and 70% (w/w), preferably about 50% (w/w), of copper diammonium chelate;
- between 5 and 20 % (w/w), preferably about 10% (w/w), of zinc diammonium chelate;
- about 1 % (w/w) of one or more fragrances;
- between 0.5 and 1 % (w/w) of one or more dispersants; and
- up to 100% (w/w) of water.

13. The composition according to claim 12, wherein said chelate of said copper and/or zinc chelate is selected from EDTA, DTPA, HDPA, HEDTA, MGDA and GLDA.

## Patentansprüche

1. Verfahren zum Reduzieren des Risikos von Epithelinfektionen in einem in einer Behausung untergebrachten Tier, umfassend den Schritt des Ausbringens einer Kupfer- und Zinkchelate umfassenden Zusammensetzung auf eine oder mehrere Innenoberflächen der Behausung.

2. Verfahren nach Anspruch 1, wobei die Kupfer- und Zinkchelate in einer Flüssigkeit suspendiert sind.

3. Verfahren nach Anspruch 1, wobei die Kupfer- und Zinkchelate in einer Flüssigkeit mikroionisiert sind.

4. Verfahren nach einem der Ansprüche 1 bis 3, wobei die Kupfer- und Zinkchelate umfassende Zusammensetzung auf den Boden oder den Boden bedeckende Streu der Behausung ausgebracht wird.

5. Verfahren nach einem der Ansprüche 1 bis 4, wobei das Ausbringen der die Kupfer- und Zinkchelate umfassenden Zusammensetzung auf die eine oder die mehreren Innenoberflächen der Behausung durch Sprühen bewerkstelligt wird.

6. Verfahren nach einem der Ansprüche 1 bis 5, wobei die Gesamtmenge an Kupfer- und Zinkchelat in der Formulierung zwischen 40% und 60% liegt.

7. Verfahren nach Anspruch 6, wobei die Zusammensetzung zwischen 30% und 50% Kupferchelat und zwischen 5% und 20% Zinkchelat umfasst.

8. Verfahren nach einem der Ansprüche 1 bis 7, wobei das Chelat des Kupfer- und/oder Zinkchelats aus EDTA, DTPA, HDPA, HEDTA, MGDA und GLDA ausgewählt ist.

9. Verfahren nach einem der Ansprüche 1 bis 8, wobei es sich bei den Kupfer- und Zinkchelaten um Dinatrium- oder Diammoniumsalze von Kupfer(EDTA) und Zink(EDTA) handelt.

10. Verfahren nach einem der Ansprüche 1 bis 9, wobei das Kupferchelat : Zinkchelat-Molverhältnis der Kupfer- und Zinkchelate im Bereich von 5 : 1 bis 1:2 liegt.

11. Verfahren nach einem der Ansprüche 1 bis 9, wobei es sich bei dem Verfahren um ein Verfahren zur Prävention einer Epithelinfektion ausgewählt aus Mastitis, exudativer Epidermitis, Mortellaro, Huffäule, Stallhuffäule, Pododermatitis, Moderhinke und Zwischeneuterdermatitis in dem Tier handelt.

12. Zusammensetzung, umfassend
- zwischen 30 und 70 Gew.-%, vorzugsweise etwa 50 Gew.-%, an Kupferdiammoniumchelat,
- zwischen 5 und 20 Gew.-%, vorzugsweise etwa 10 Gew.-%, an Zinkdiammoniumchelat,
- etwa 1 Gew.-% eines oder mehrerer Duftstoffe,
- zwischen 0,5 und 1 Gew.-% eines oder mehrerer Dispersionsmittel und
- bis zu 100 Gew.-% an Wasser.

13. Zusammensetzung nach Anspruch 12, wobei das Chelat des Kupfer- und/oder Zinkchelats aus EDTA, DTPA, HDPA, HEDTA, MGDA und GLDA ausgewählt ist.

## Revendications

1. Procédé pour la réduction du risque d'infections épithéliales chez un animal gardé dans un logement, comprenant l'étape d'application d'une composition comprenant des chélates de cuivre et de zinc à une ou plusieurs surfaces intérieures dudit logement.

2. Procédé selon la revendication 1, lesdits chélates de cuivre et de zinc étant en suspension dans un liquide.

3. Procédé selon la revendication 1, lesdits chélates de cuivre et de zinc étant micronisés dans un liquide.

4. Procédé selon l'une quelconque des revendications 1 à 3, ladite composition comprenant des chélates de cuivre et de zinc étant appliquée sur le sol ou le revêtement de litière dudit sol dudit logement.

5. Procédé selon l'une quelconque des revendications 1 à 4, l'application de la composition comprenant des chélates de cuivre et de zinc sur ladite ou lesdites surfaces intérieures dudit logement étant réalisée par pulvérisation.

6. Procédé selon l'une quelconque des revendications 1 à 5, la quantité totale de chélates de cuivre et de zinc dans ladite formulation étant comprise entre 40 % et 60 %.

7. Procédé selon la revendication 6, la composition comprenant entre 30 % et 50 % de chélate de cuivre et entre 5 % et 20 % de chélate de zinc.

8. Procédé selon l'une quelconque des revendications 1 à 7, ledit chélate dudit chélate de cuivre et/ou de zinc étant choisi parmi l'EDTA, le DTPA, l'HDPA, l'HEDTA, le MGDA et le GLDA.

9. Procédé selon l'une quelconque des revendications 1 à 8, lesdits chélates de cuivre et de zinc étant des sels disodiques ou de diammonium de cuivre(EDTA) et de zinc(EDTA).

10. Procédé selon l'une quelconque des revendications 1 à 9, le rapport molaire chélate de cuivre : chélate de zinc desdits chélates de cuivre et de zinc se situant dans la plage de 5 : 1 à 1 :2.

11. Procédé selon l'une quelconque des revendications 1 à 9, ledit procédé étant un procédé de prévention d'une infection épithéliale choisie parmi la mastite, l'épidermite exsudative, la dermatite digitale, la pourriture du sabot, la pourriture du sabot stable, la pododermatite, le piétin et la dermatite de la fente du pis chez ledit animal.

12. Composition comprenant
- entre 30 et 70 % (p/p), préférablement environ 50 % (p/p), de chélate de cuivre diammonium ;
- entre 5 et 20 % (p/p), préférablement environ 10 % (p/p), de chélate de zinc diammonium ;
- environ 1 % (p/p) d'une ou plusieurs fragrances ;
- entre 0,5 et 1 % (p/p) d'un ou plusieurs dispersants ; et
- jusqu'à 100 % (p/p) d'eau.

13. Composition selon la revendication 12, ledit chélate dudit chélate de cuivre et/ou de zinc étant choisi parmi l'EDTA, le DTPA, l'HDPA, l'HEDTA, le MGDA et le GLDA.
